# EUROPEAN PATENT APPLICATION

(11) **EP 3 357 997 A1**
(43) Date of publication of application: **08.08.2018**
(21) Application number: 16793933.9
(22) Date of filing: 28.09.2016
(51) Int. Cl.: C12M 1/12, G01N 33/48

(54) **INTEGRATED FILTER-HOLDER AND MICROORGANISM CONCENTRATION AND DETECTION METHOD**

(30) Priority: 01.10.2015 ES 201531409
(71) Applicant: Consejo Superior De Investigaciones Científicas (CSIC), 28006 Madrid (ES); Institut D'investigació Germans Trias I Pujol, 08916 Badalona (Barcelona) (ES); Centro de Investigación Biomédica en Red (CIBER), 28029 Madrid (ES); Universitat Autònoma de Barcelona, 08193 Bellaterra (ES)
(72) Inventor: CASTILLO FERNÁNDEZ, Óscar, 08193 Cerdanyola del Vallès (Barcelona) (ES); MUÑOZ PASCUAL, Fco. Javier, 08193 Cerdanyola del Vallès (Barcelona) (ES); URIA MOLTÓ, Naroa, 08193 Cerdanyola del Vallès (Barcelona) (ES); PARRAGA NIÑO, Noemí, 08916 Badalona (Barcelona) (ES); SABRIA LEAL, Miquel, 08916 Badalona (Barcelona) (ES)
(74) Representative: Pons
(86) International application number: PCT/ES2016/070680
(87) International publication number: WO 2017/055662

(57) **Abstract**

An integrated filter-holder that enables a target microorganism of a fluid to be concentrated through the filtration of said fluid and a culture of said target mechanism to enable the detection thereof. The filter-holder comprises a top piece (1), an intermediate piece (5) and a bottom piece (4) joined together. A filtration membrane is arranged on the intermediate piece (5) and there is a reaction chamber (6) over the former in which the cultivation of the microorganism trapped in the filtration membrane when the sample is filtered takes place. The filtration membrane is kept inside the device during the entire concentration and detection method. An object of the invention is also the concentration and detection method of microorganisms in a filter-holder such as the one described above.

## Description

### OBJECT OF THE INVENTION

The present invention falls within the technical field of filtering equipment for fluids. It also includes the analysis and detection of microorganisms in the same device.

More specifically, an integrated filter-holder is described that enables both the concentration and detection of the microorganisms concentrated by filtration to be carried out in the device itself whilst staying leak-tight during the concentration and detection.

### BACKGROUND OF THE INVENTION

There is a clear need to generate new devices for detecting and controlling certain microorganisms in different environments. The main requirement of the market consists in that the new systems developed must be simple, portable and have a low manufacturing cost and low analysis cost, as well as enabling fast detection (to the order of hours) with the aim of having effective control, via the proliferation of the target microorganism.

In recent years various detection systems based on immunological methods on paper have appeared. The paper used is normally cellulose or other derivatives. These devices may be classified by the sample processing mode in: lateral flow systems or static systems that use processes where paper is immersed in different reagents. Systems that emulate a well array that may be used directly in an enzyme-linked immunosorbent assay, via hydrophobic substance primers to delimit the area of action, are also known.

In general, all these systems use the absorption capacity of the cellulose to contain reagents or the biomolecules responsible for detection, inside the paper. In this way, the reagent costs and volumes are decreased. The majority of these systems tend to be supplied with pre-loaded reagents.

Normally, the detection limit required by the control system is very low, i.e. it corresponds to a detection of very few cells per volume, which leads to the need to process large sample amounts. In order to avoid the detection limitations of the sensor systems, in the relationship between its sensitivity and processed sample volume, it is necessary to concentrate the sample before the measurement. The most common method for carrying out this concentration is mechanical filtration of the sample, due to its low cost, efficiency and speed.

In the field of filtration there are a variety of known systems, materials and types of filtration that enable bacteria to be concentrated. It is essentially a surface or three-dimensional structure that enables the different elements contained in the initial liquid solution to be blocked. There are encapsulation systems that enable bacteria to be incubated and liquids to be filtered in a single system.

Several types of filters are also known in the state of the art that additionally enable microorganisms to be detected by their growth and development in culture mediums.

For example, document WO9947637 describes a microbiological testing device for a liquid sample comprising a top piece and a bottom piece joined together with a membrane therebetween. The reagent is found in the bottom piece, and the device is turned over so that the reagent acts on the microorganisms that remain in the membrane when filtration is carried out. In this way, the reagent passes through the membrane and passes to the top portion (which is now at the bottom). This device and the technical characteristics thereof are limited to the use of a flexible membrane. The device described is intended for its inclusion in a larger container in which the sample analysis is carried out.

Document EP0832181 also describes different solutions to prevent the filtration membrane from being contaminated by other microorganisms that are to be detected.

The need therefore arises to develop microorganism detection systems in water-cooled systems, from air conditioning to cooling towers of any scale, which may potentially generate an outbreak of *Legionella.* In these refrigeration systems there is a proliferation of microorganisms due to the water temperatures. In the case of *Legionella,* it is worrying because it can survive in aerosols and be released into the environment and can affect the population by causing pneumonia. Moreover, there is currently a need for this type of technology in other sectors, such as the food, environmental and health industry sectors.

### DESCRIPTION OF THE INVENTION

The present invention presents an integrated filter-holder that enables both the concentration of microorganisms, object to be studied, and the analytic measurement using affinity detection methods using recognition proteins such as antibodies in so-called immunological methods, to be carried out in the same device. That is to say, mechanical filtration of the sample to concentrate the biological material on the filtration membrane is carried out in the filter-holder and a culture is carried out in order to, through methods based on the affinity of biomolecules, carry out a microbiological detection.

The filter-holder comprises a top piece, an intermediate piece and a bottom piece, where said pieces may be separated from each other in an embodiment wherein the filter-holder may be reused or may be fixedly joined together in an embodiment wherein the filter-holder is single use.

In the exemplary embodiment wherein the filter-holder is reusable, the filtration membrane is arranged therein and once the filtration and the culture have been carried out, the filter-holder may be opened, the membrane removed and changed for a new one. In the exemplary embodiment wherein the filter-holder is disposable, all the pieces thereof are integrally joined to each other and when the filtration and culture have been carried out, it is simply disposed of and a new filter-holder is used in order to carry out a new filtration.

The functions that the integrated filter-holder of the present invention must fulfil are:
- filtration must be leak-tight and the fluid must pass entirely through the filtration membrane to guarantee that the retention percentage of the microorganism under study is the highest possible,
- enable the hybridisation between the membrane protein of the microorganism and the recognition molecule (such as, for example, antibody, peptide, etc.) for which it comprises an incubation chamber,
- enable the exchange of different solutions without the extraction of the filtration membrane to be able to carry out different stages of the detection by affinity.

The microorganisms that are in the sample are retained in the filtration membrane when it is mechanically filtered. The filter may be a mesh structure such as PVDF (polyvinylidene fluoride) or nitrocellulose, or a micro-perforated surface such as polycarbonate. Before the arrangement of the membranes on the filter-holder, a pre-treatment is applied to them to reduce the non-specific binding of the detection proteins, but without affecting the porosity thereof. Before this, affinity detection is carried out in the filter-holder itself by inserting the corresponding reagents into a reaction chamber of the filter-holder (which is arranged immediately over the membrane) so that they react with the microorganisms retained in the membrane. As such, the membrane catches the bacteria (microorganisms) therein or on the surface thereof and the chemical recognition reaction that enables sensing takes place on the same.

Preferably, the system is designed to carry out an indirect measurement, for example, in the case of carrying out immunological detection, the antibody concentration in the filtration membrane is detected. The measurement of the final concentration of the product obtained by the reaction is directly related to the concentration and presence of microorganisms trapped in the filtration membrane. Based on the measurement of antibodies, the amount of bacteria detected in the filter can be obtained and by extrapolating the results, the contamination of the body of water or corresponding system from which the sample was taken is calculated.

The detection reaction is a reaction that is catalysed by a detection element conjugated with a recognition protein. The result and efficiency of this reaction may be measured. The result of this reaction is proportional to the presence in concentration of the detection element, which in turn is proportional to the amount of recognition protein that has reacted with the microorganisms that are present in the membrane. In this way, the quantification is carried out via an indirect measurement of the presence of microorganisms that are sought to be detected in the membrane.

The recognition elements have elements conjugated in the structure thereof which react with a specific solution that enables it to be detected and quantified through the measurement of the by-product of this reaction. For example, the enzyme HRP (horseradish peroxidase) may be conjugated to the structure of an antibody to determine the amount of antibody that is found in the membrane due to the effect of the enzyme as an oxidant of a given substrate. These changes are subsequently detected via electrochemical or optical techniques.

The filter-holder comprises a top piece through which the sample and reagents are inserted, an intermediate piece in which the reaction chamber is found, a bottom piece through which the filtered sample is released and a membrane that is housed between the intermediate piece and the bottom piece. The top, intermediate and bottom pieces are removeably or fixedly joined to each other (removeably in the case of reusable filter-holders and fixedly in the case of disposable filter-holders).

In the intermediate piece there is a reaction chamber, which is arranged immediately above the filtration membrane. The detection of the microorganisms retained in the filtration membrane takes place in said reaction chamber.

In an exemplary embodiment, the filter-holder may further comprise a diffuser which is arranged in the intermediate piece, above the reaction chamber. The diffuser is a piece with a plurality of holes that enable the sample to be homogeneously distributed over the entire surface of the filtration membrane. The sample passes from an inlet hole of the top piece, through which it enters the filter-holder, to the diffuser where it is distributed over the entire surface thereof to pass to the filtration membrane, uniformly for the entire surface of said filtration membrane.

The top piece may have one or more upper holes. The number of upper holes depends on the type of membrane and entrapment in the membrane. For hydrophobic filters with a perforated surface (such as polycarbonate for example), the microorganisms are well retained and fixed on the surface of the filtration membrane and therefore two holes in the top piece are necessary. For membranes in the form of mesh, with a 3D structure (for example nitrocellulose or PDVF). The top piece preferably comprises a single upper hole such that the different reagents that are used in the process since the microorganisms are retained inside the mesh.

One advantage associated with the filter-holder of the present invention is that by the reaction chamber itself being comprised therein arranged on the filtration membrane, the detection operation that is carried out just after the filtration operation, is carried out inside the device itself without removing the filtration membrane. In this way, the membrane is kept leak-tight in the intermediate piece such that the culture cannot be contaminated.

The bottom piece comprises a lower hole intended for the outlet of the sample after having been filtered in the filtration membrane.

The at least one upper hole (in the top piece) and the lower hole (in the bottom piece) may be closed, for example via covers that form part of the filter-holder itself for carrying out the detection protocol.

In the embodiment in which the filter-holder is reusable, it must be assembled with the filtration membrane therein. In order to do so, the bottom piece is arranged, on it is placed the intermediate piece and the filtration membrane is placed thereon. Subsequently, the top piece is placed and all the pieces are joined together, preferably via screws. Also preferably, the filter-holder comprises gaskets that are arranged between the top piece and the intermediate piece and between the intermediate piece and the bottom piece. These gaskets ensure the leak-tightness inside the filter-holder.

The filtration membrane is a porous membrane and preferably has a pore size of 0.2 µm to 0.45 µm which enables submicronic particles such as microorganisms or other biological samples such as proteins to be retained. The different treatments on the membranes are carried out via different coatings of porous membranes to guarantee the retention of bacteria (microorganisms) in the membranes as well as preventing the adhesion of other elements that may alter the results of said detection.

The filter-holder of the present invention is simpler than other devices of the state of the art since it does not comprise additional elements therein to house the reagents during filtration. The filter-holder is designed to insert the reagents in the same reaction chamber in which the sample is previously inserted. All the reagents are inserted directly into the chamber. This reaction chamber is arranged in the intermediate piece of the filter-holder on the filtration membrane. The reagent is inserted through the same upper hole as the sample and follows the same path to the filtration membrane.

The reaction chamber of the filter-holder has to enable the reagents to mix. By using the peristaltic pump to insert the sample and the reagents in said reaction chamber, fluid movements are generated, thus creating convections that guarantee the mixing of the sample and reagents, as well as washing the recognition protein. This washing and transportation process enables the elimination of excess recognition protein that has not reacted with the microorganisms present in the membranes.

### DESCRIPTION OF THE DRAWINGS

As a complement to the description being made, and for the purpose of helping to make the characteristics of the invention more readily understandable, in accordance with a preferred example of practical embodiment thereof, said description is accompanied by a set of drawings constituting an integral part thereof which, by way of illustration and not limitation, represent the following:
Figure 1a.- Shows a cross-sectional view of the filter-holder where the top piece, intermediate piece and bottom piece may be seen in a top elevation view.
Figure 1b.- Shows a view of the top piece of the filter-holder.
Figure 1c.- Shows a view of the intermediate piece of the filter-holder.
Figure 1d.- Shows a view of the bottom piece of the filter-holder.
Figure 2a.- Shows a graph representing the retention results obtained in a retention study of *E*. *coli* in pump filtration, syringe filtration and filtration with the integrated filter-holder of the present invention.
Figure 2b.- Shows a graph representing the retention results obtained in a retention study of *L. Pneumophila* in pump filtration, syringe filtration and filtration with the integrated filter-holder of the present invention.
Figure 3.- Shows a graph representing the absorbency obtained via enzyme-linked immunosorbent assay (ELISA), using a specific antibody against *E.coli,* marked with the enzyme HRP (*horseradish peroxidase*), measuring the antibody amount in the sample, linking the effect of the enzyme HRP on the substrate TMB *(tetramethylbenzidine)* for a nitrocellulose filtration membrane without blocking, a chemical blocking and an organic blocking.
Figure 4.- Shows a calibration curve that links the detection capacity of the filter-holder with absorbency values at 480 nm after filtering different concentrations of *E.coli* on PVDF filtration membranes blocked before filtration and after filtration with Tween20 at 1%.
Figure 5.- Shows a calibration curve that links the absorbency at 480 nm measured after filtering different concentrations of *Legionella* on nitrocellulose filters blocked with Tween20 at 1% with the integrated filter-holder of the present invention.
Figure 6.- Shows a graph representing the absorbency of water samples in the integrated filter-holder of the invention with a nitrocellulose filtration membrane with Tween20 blocker and specific antibodies for *Legionella pneumophila* conjugated with HRP and it may be seen that the results are different for the two samples thus enabling a sample containing *Legionella pneumophila* and one that does not to be distinguished.

### PREFERRED EMBODIMENT OF THE INVENTION

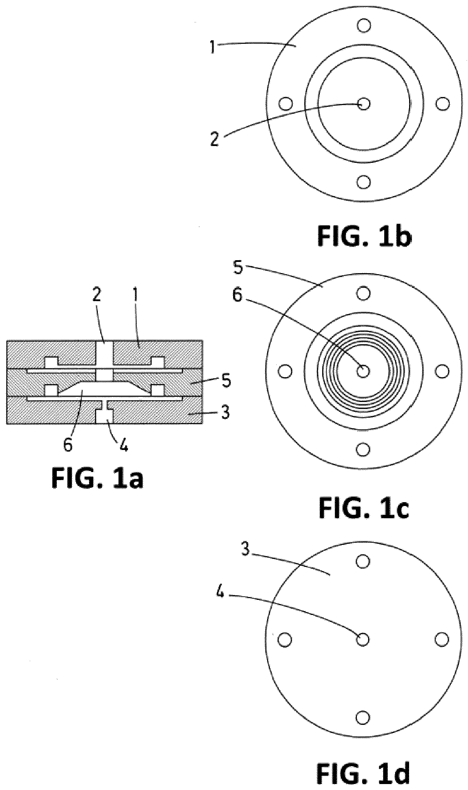

What follows is a description, with the help of figures 1 to 6, of exemplary embodiments of the invention.

An integrated filter-holder which enables the filtration and cultivation of a sample to be carried out is described. It enables the concentration of a microorganism under study (thanks to the filtration of the sample) to be carried out in the device itself and the cultivation of said microorganism for the detection thereof, for example, via immunological detection. The great advantage of the present invention is that it enables the two operations to be carried out without handling the membrane, thus guaranteeing correct detection.

The filter-holder comprises a top piece with at least one upper hole for the inlet of the sample and a bottom piece with at least one lower hole for the outlet of the now filtered sample. It likewise comprises at least one filtration membrane which is preferably a filtration membrane that is a porous membrane, whether made of a polymer such as PVDF (polyvinylidene fluoride), polycarbonate or materials derived from paper such as nitrocellulose. Preferably, the size of the pores of the filtration membrane is between 0.10 µm and 0.45 µm. Preferably, the size of the pore is 0.20 µm. Detergents such as Tween®, organic molecules such as *BSA* (Bovine Serum Albumins) and in general any substance used as a blocker in immuno-detection protocols may be used as blocker substances. The filtration membrane may be, for example, made of PVDF (polyvinylidene fluoride), nitrocellulose, fibreglass and polycarbonate.

The most important technical characteristic of the filter-holder of the present invention is that it comprises an intermediate piece, arranged between the top piece and the bottom piece and joined to each other, and which is intended to house the filtration membrane and a reaction chamber. Said reaction chamber is a hollow space, arranged above the filtration membrane and which is intended to receive a solution and reagent during the cultivation process of the microorganism trapped in the filtration membrane.

The reaction chamber is connected to the upper hole for the inlet of the sample and of the solution and the reagent when appropriate. Preferably, said reaction chamber which is arranged over the filtration membrane, has a volume of less than 3 ml and more preferably between 1 ml and 2 ml.

In an exemplary embodiment the top piece comprises two upper holes that open into the reaction chamber. In the case of polycarbonate filtration membrane, the top piece comprises two upper holes due to the fact that the bacteria are trapped in the surface of the membrane. In this way, the reagents enter and are released through the top portion of the system. The washing solution is a PBS and Tween® solution, whilst the reagent solutions tend to be hydrogen peroxide solutions. In this exemplary embodiment, one of the upper holes is intended for the insertion of the sample, the solution and the reagent into the reaction chamber. The other upper hole is intended for the extraction of the solution and the reagent that were previously inserted in the filter-holder.

Also preferably, the filter-holder comprises a cover linked to each one of the holes that enables the inner space of the filter-holder to be closed. That is to say, when the covers are closed, the filtration membrane and the reaction chamber (with the solution or the reagent, as appropriate) are kept isolated during detection in order to prevent possible contamination of the reagent. Moreover, at least the lower hole must be closed in order to prevent the solution or reagent that is inserted through one of the upper holes from passing through the filtration membrane and being released through said lower hole.

In an exemplary embodiment, the filter-holder is reusable. In this embodiment, the pieces of the filter-holder may be separated to enable the user to remove the filtration membrane when it has been used for a filtration and a cultivation. This filtration membrane must simply be replaced by a new one to once again use the filter-holder. Preferably, the top piece, the intermediate piece and the bottom piece are joined to each other with through screws. These screws enable the pieces to be kept joined together during the concentration and detection method and subsequently be separated to change the filtration membrane.

The non-specific binding of the reagents to the filtration membrane is prevented via the pre-treatment of said filtration membrane and a treatment after hybridisation with known substances with very low non-specific reactivity. Preferably, the pre-treatment of the membrane is carried out with the same blocking components that may be used in washing, such as BSA, Tween®, Bloking®, etc. for example.

Said pre-treatment is carried out by absorption of the non-specific, low-reactivity substances in the filtration membrane structure or in the surface thereof (depending on the substance). The treatment of the filtration membrane after hybridisation is carried out via the slight movement of a solution over the filter to wash and remove the antibody spoil.

In order to guarantee the leak-tightness in the filter-holder, it preferably comprises a first sealing gasket between the top piece and the intermediate piece and a second sealing gasket between the intermediate piece and the bottom piece.

In another exemplary embodiment, the filter-holder may be single use in which case the top piece, the intermediate piece and the bottom piece are integrally joined together. In this case, the user directly disposes of the filter-holder and if desired a new filtration and cultivation is carried out using a new filter-holder. It is made of Metacrlilato®, Nylon® or thermoplastics.

The filter-holder may further comprise a diffuser arranged in the intermediate piece. The diffuser comprises a plurality of holes and is configured to distribute the inlet flow from the upper hole towards the filtration membrane.

An object of the present invention is also a microorganism concentration and detection method of a sample in an integrated filter-holder as described above.

The inlet flow in the filter-holder throughout the method changes since the sample under study is first inserted and then a solution and subsequently a reagent are inserted. The outlet flow is each one of them as appropriate.

The method proposed comprises the following stages:
- insert the sample through the top piece (1) making the sample pass through the filtration membrane arranged in the intermediate piece (5) such that the particles under study are retained in the filtration membrane,
- extract the filtered sample through the bottom piece (3),
- insert a solution through the top piece (1) into the reaction chamber (6) and keep it in the reaction chamber (6) for a predetermined period of time until it has reacted with the particles retained in the filtration membrane,
- extract the solution through the bottom piece (3),
- insert a reagent through the top piece (1) into the reaction chamber (6) and keep it in said reaction chamber (6) until it has reacted with the mixture of the solution and particles retained in the filtration membrane,
- extract the reagent through the bottom piece (3).

As described above, the filter-holder may have one or two upper holes (2) in the top piece (1). In the exemplary embodiment wherein the filter-holder comprises two upper holes in the top piece, the concentration and detection method for microorganisms in the filter-holder of the present invention comprises the following stages:
- insert the sample through at least one upper hole (2) of the top piece (1), the second upper hole (2) remains closed, such that the microorganisms in the sample are retained in the filtration membrane when they pass through the membrane and the now filtered sample is released through the lower hole (4) of the bottom piece (3),
- close the lower hole (4),
- insert a solution comprising a recognition protein through the other upper hole (2) whilst the upper hole through which the sample was inserted remains open to enable the reaction chamber (6) to be filled,
- close the upper holes (1, 2) to keep the solution in the reaction chamber,
- keep the filter-holder closed for a predetermined period of time until the solution reacts with the microorganisms that are trapped in the filtration membrane,
- open an upper hole (2) and remove the inserted solution,
- apply a washing reagent through an upper hole (2), keeping the other upper hole (2) open and the lower hole (4) closed to drag the recognition protein that has not reacted with the microorganism and remains free in the solution,
- insert a reagent containing an enzyme substrate which reacts with the recognition protein, through an upper hole (2) with the other upper hole (2) open and the lower hole (4) closed such that the reagent remains in the reaction chamber (6),
- keep for a predetermined period of time until the reagent reacts with the catalyst element of a reaction (enzyme) conjugated in the reaction protein or until it reacts,
- open an upper hole (2) or the lower hole (4) and remove the inserted reagent.

In the exemplary embodiment wherein the filter-holder comprises one upper hole (2) in the top piece (1), the microorganism concentration and detection method in the filter-holder of the present invention comprises the following stages:
- insert the sample through the upper hole (2) of the top piece (1), such that the microorganisms in the sample are retained in the filtration membrane and the filtered sample is released through the lower hole (4),
- insert a solution with a recognition protein through the upper hole (2) whilst the lower hole (4) remains closed to enable the reaction chamber (6) to be filled,
- close the upper hole (2) to keep the solution in the reaction chamber (6),
- keep the solution in the reaction chamber (6) for a predetermined period of time until the solution reacts with the microorganisms that are trapped in the filtration membrane,
- open the upper and lower holes (2, 4) and remove the solution,
- apply a washing reagent through the upper hole (2), with the lower hole (4) open to drag the recognition protein that has not reacted with the microorganism and remains free in the solution,
- insert a reagent containing an enzyme substrate that reacts with the recognition protein, through the upper hole (2) with the lower hole (4) closed such that the reagent remains in the reaction chamber,
- keep for a predetermined period of time until the reagent reacts with the catalyst element of a reaction (enzyme) conjugated in the reaction protein or until it reacts,
- open the upper hole (2) and the lower hole (4) and remove the inserted reagent.

The pieces of the filter-holder are never separated throughout the method and the filtration membrane is not removed. Therefore, the method is entirely carried out in a leak-tight manner which guarantees correct filtration and incubation (external contamination is prevented).

When this method has ended, the final solution as a result of the last reaction is extracted and analysed via electro-chemical or colorimetric methods.

Preferably, the sample is inserted in the filter-holder via a peristaltic pump that enables the sample to be forced to pass through the filtration membrane. Depending on the pathogen and the sample and the contaminations of inorganic elements (salts, metal oxides, solid materials), different sample flows and pressures are used during the filtration process in order to have optimal filtration results. At this point additional filtration stages may also be included to remove solid materials in suspension, via nitrocellulose, PVDF (polyvinylidene fluoride) or polycarbonate membranes with pores of 5 to 20 µm depending on the contaminations.

In an exemplary embodiment of the invention, the filter-holder is connected by the upper hole to the container containing the sample via a peristaltic pump that inserts the sample into the filter-holder with a suitable flow and pressure. The sample enters the filter-holder under said flow and pressure conditions and is forced to pass through the filtration membrane. The lower hole, arranged in the bottom piece, is intended for the now filtered sample to pass through (it enables said filtered sample to be released from the filter-holder).

In an exemplary embodiment, the filter-holder of the present invention is capable of filtering up to 700 ml/h and a flow of 1.5 ml/sec.

The filtration membrane that is in the filter-holder depends on the microorganism that is to be studied. Depending on the microorganism under study, the membrane is prepared to trap it when the sample is filtered. Once the sample has been completely filtered and the target microorganisms have been trapped in the membrane, there is a stage for closing the lower hole. The solution must subsequently be inserted through the upper hole through which the sample was previously inserted. Since the lower hole is closed, the solution is trapped in the reaction chamber over the filtration membrane. When the solution has been inserted, the upper hole must be closed and the filter-holder kept closed during a specific period of time to enable the solution and microorganisms in the filtration membrane to interact.

After a predetermined time has passed, preferably between 30 and 50 minutes, the solution must be extracted. This extraction may be carried out through an upper hole (in the exemplary embodiment wherein the filter-holder comprises more than one upper hole) or may be carried out through the lower hole.

After the previous stage, a washing stage may be carried out to remove the spoil of the solution that remains inside the filter-holder. For example, this washing may be carried out with PBS and Tween® as described above.

Subsequently, the lower hole must be closed again in the event that it has been opened in the previous stage and a reagent is inserted through the upper hole. After this stage, the upper hole must be closed in order for the reagent that is in the reaction chamber to be able to react with the microorganism and with the solution that has been obtained in the previous stages. The reagent is kept inside the filter-holder for a period of time not greater than 20 minutes.

Once the predetermined time has passed, the reagent must be extracted through the lower hole or through the other upper hole in order to analyse the solution that has been obtained. When it is analysed, an indirect measurement is obtained of the microorganisms that are present in the sample based on the antibodies (in the case of immunological detection) that have appeared. Preferably, electrochemical measurements are used for this analysis.

### Exemplary embodiments

### Example 1:

A leak-tight and filtration efficiency test has been carried out on the integrated filter of the present invention via the use of *Escherichia coli* and *Legionella pneumophila* as target microorganisms (microorganisms under study). As such, based on a controlled laboratory culture, three solutions with different concentrations are prepared: 1e6, 1e4 and 1e2 ufc in 50 ml of water, and the ratios of retention efficiency were studied depending on the amount of bacteria present in the sample. This efficiency was also compared to different commercial filtration systems and membranes of different materials.

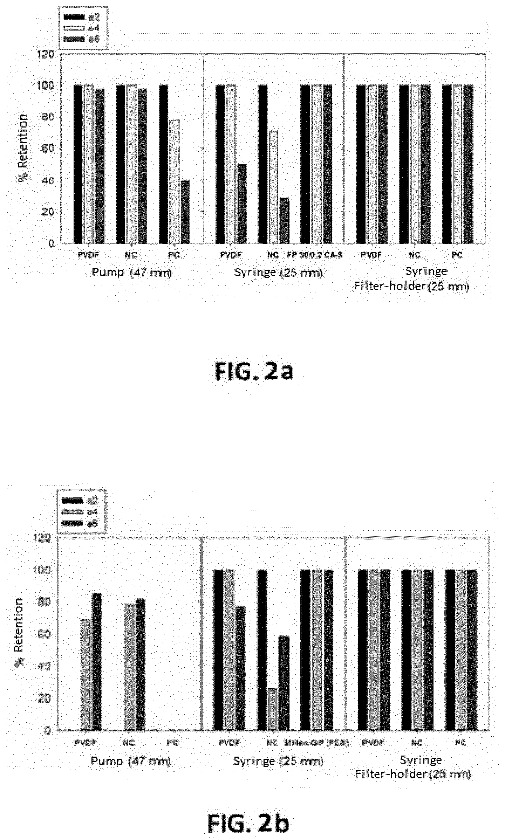

Figure 2 shows the results of filtration efficiency for two commercial filtration systems (pump filtration with 47 mm diameter filters and syringe filtration with 25 mm diameter filters) and for the integrated filter-holder of the present invention with a membrane made of nitrocellulose, PVDF, polycarbonate and encapsulated commercial filters

Figure 2a represents the retention values for *E*. *coli* whilst the results for *L. pneumophila* are shown in Figure 2b. In general, both bacterial species show differences in retention according to the type of filtration and membrane material used. However, there are only two mechanisms that enable 100% filtration efficiency, for both *E. coli* and *L. pneumophila,* regardless of the membrane material used: syringe filtration with encapsulated filters and the integrated filter-holder of the present invention.

The first results were obtained by using a commercial SWINNEX® system for 47 mm membranes. The second results were obtained by using a commercial SWINNEX® system for 25 mm membranes and an encapsulated and leak-tight filtration system. The third results were obtained by using the filter-holder of the invention with 25 mm membranes. The filtration method with the filter-holder of the invention is also carried out with a syringe. The best results from commercial systems correspond to membranes that are sold encapsulated, without threads and heat-sealed. The filter-holder of the present invention enables the same level of efficiency to be reached.

### Example 2:

A study was carried out on the minimisation of non-specific binding between antibody and membrane via the use of albumin as an example of an organic blocker and Tween20 as a chemical blocker.

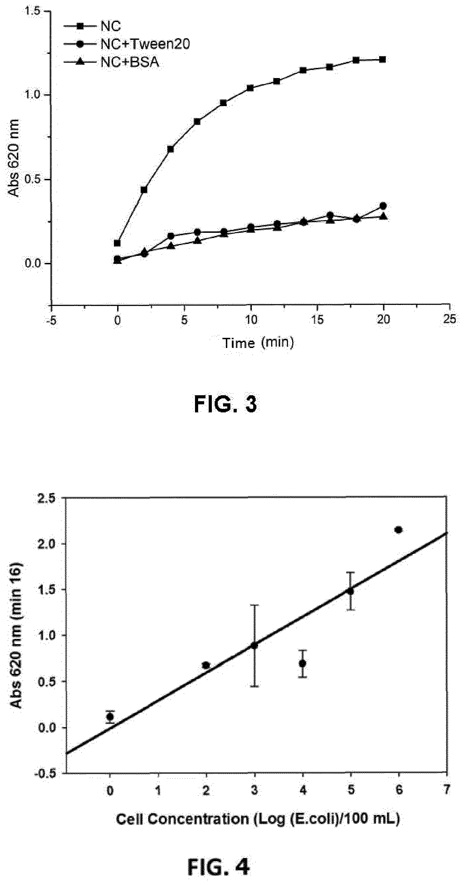

The results corresponding to this example have been represented in figure 3. Said figure shows a graph representing the absorbency obtained via enzyme-linked immunosorbent assay (ELISA), using a specific antibody against *E.coli,* marked with the enzyme HRP, measuring the antibody amount in the sample, linking the effect of the enzyme HRP on the substrate TMB. The line with squares is the result obtained using a nitrocellulose filter without a blocker. The lines with triangles and circles represent the results obtained by using a chemically blocked nitrocellulose filter (Tween20 line with circles) and another organically blocked filter (BSA line with triangles). The graph shows that by using these blockers, the signal obtained is much lower than for the case of the raw membrane, thus minimising the specific binding between the antibody and nitrocellulose membrane.

The membrane material used for the blocking study was nitrocellulose disks of 0.25 cm2 with a pore size of 0.2 micron. For the study of the efficiency of chemical and organic compounds in minimising the non-specific binding between the antibody and membrane, Tween®20 and albumine (BSA) are used as examples of both types, respectively. Likewise, in this test an anti-E.coli antibody marked with the enzyme peroxidase (HRP) was used.

In this way, two filtration membranes were incubated in both tubes that contained, in one case 500 µl of Tween®20 and in the other 500 µl of BSA, both in a final concentration of 1% in phosphate buffered saline. Additionally, a third filter was inserted in a tube with 500 µL of phosphate buffered saline without any type of blocker compound such as non-blocked blank membrane.

After one hour of contact between the membrane and each one of the blockers, these were extracted from the tubes and washed three times by submerging them in a solution formed again of phosphate buffered saline supplemented with detergent Tween®20 at a concentration of 0.5% to remove the antibody present in the filter but not absorbed.

Once the washing has been carried out, the antibody that is non-specifically bound to the filtration membrane is determined via colorimetric techniques. The filters were inserted in a standard plaque used for enzyme-linked immunosorbent assay (ELISA). After this, 100 µl of enzyme substrate TMB per filter was added was added and a mechanism measurement is carried out in an ELISA plaque detector for 20 minutes measuring absorbency at 620 nm to obtain a reaction mechanism between the enzyme HRP (which is bound to the antibody) and the substrate (TMB).

The results that are presented in figure 3 show a much higher absorbency for the non-blocked nitrocellulose filter (NC), indicating much greater binding of the antibody in this filter. Additionally, there were no large differences between both chemical and organic blockers, which means both may be used with good results to minimise non-specific binding.

Said figure 3 shows a study of non-specific binding of the antibody to nitrocellulose membranes treated via detergents and proteins, and untreated membranes. The graph represents the results obtained via colorimetric measurements, using HRP and TMB solution as a reaction substrate. It can be seen in this graph how the treatment efficiently reduces the non-specific absorption of the antibody in the membrane.

### Example 3:

To check the possibility of detecting different types of microorganisms. The operation of the filter-holder compared to several types of microorganisms such as *E.coli* and *Legionella* was tested.

In the case of E.coli, polycarbonate membranes of 25 mm diameter blocked with PBS-Tw20 at 1% before and after were used. Different suspensions of *E.coli* at a final concentration of 10², 10³, 10⁴, 10⁵, 10⁶ total cells in 100 ml, making solutions of a bacterial culture of 10⁸ cells, were filtered at a flow of 0.5 ml/s.

Once the filtration was completed, four perforations were carried out in each filter to collect samples that were 5 mm in diameter. Three of them were used as replicas to analyse the binding of the antibody to the filter via incubation with antibodies. The fourth sample is used as a blank by incubating it without antibodies. After the incubation with the antibody, three 5-minute washes were carried out by inserting the membranes in a solution of Tween® 20 and PBS. Lastly, the filters were placed on ELISA plaques with 96 wells and 100 µl of TMB was added to them in order to be able to measure the reaction mechanism at a wavelength of 620 nm for 20 mins. The calibration curve was carried out using the absorbency data collected at minute 16 of the reaction and is shown in figure 4.

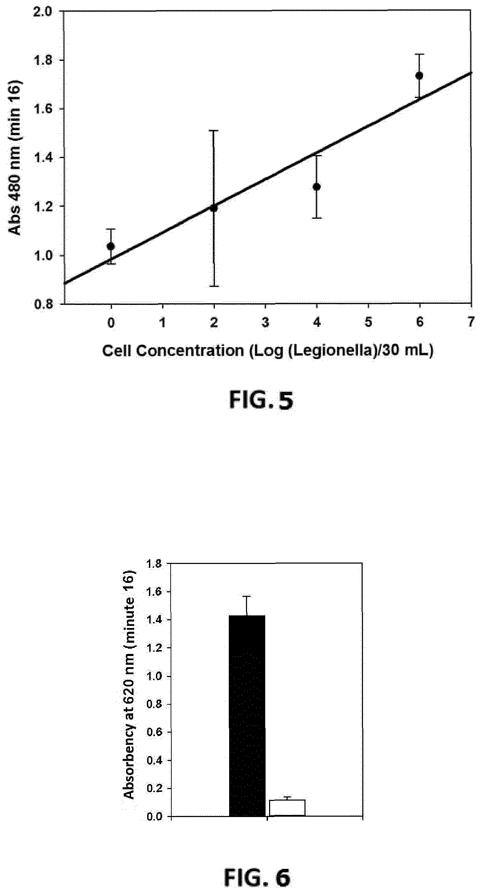

Said figure shows the absorbency at 620 nm, measured after filtering different concentrations of *E.coli* over PC filters blocked with Tween® 20 at 1%. In the case of *Legionella,* the same method as above was used but in this case with nitrocellulose filters blocked with Tween® 20 before the sample is filtered. In this case, total concentrations of 10⁶, 10⁴ and 10² *Legionella* in 30 ml were filtered with a flow of 0.5 ml/s. The results are presented in figure 5. The measurements for this example were carried out in the absorbency at 480 nm.

Figure 5 shows the calibration curve that links the absorbency at 480 nm measured after filtering different concentrations of *Legionella* on nitrocellulose filters blocked with Tween20 at 1%.

### Example 4:

Measurements with real samples from industrial facilities were carried out to detect *Legionella.*

The two samples to be analysed were water from two different areas of large-scale facilities, one with frequent presence of *Legionella* and other lacking *Legionella.*

The membranes used for the filtration were nitrocellulose disks with a pore size of 0.2 micron and 26 mm in diameter, blocked with Tween®20 at a concentration of 1% in order to prevent non-specific binding between the antibody and the membrane. After the filtration of both samples and a blank consisting of sterile water (without microorganisms), incubation with *Legionella* antibodies marked with HRP was carried out in order to also repeat an optical detection.

Figure 4 represents the absorbency measured at a wavelength of 620 nm at minute 16 during a reaction mechanism of 20 minutes between the colorimetric substrate (TMB) and the filter. These results are normalised by the absorbency measured in the filters used for the water that did not contain bacteria. As such, the graph shows how sample 1, which came from the area frequently contaminated with *Legionella,* gives a much higher signal than sample 2, which comes from an area with no contamination, and had values close to 0.

Figure 6 represents a study on the absorbency of real water samples of two sanitary water facilities from different changing rooms of an industrial plant in Tarragona. Two samples were taken, M1 from a facility that usually gives a positive result for *Legionella pneumophila,* and M2 from a facility with no presence of bacteria, using the filter-holder of the present invention and specific antibodies for *Legionella pneumophila.* The results obtained via colorimetric measurements, using HRP and TMB solution as a reaction substrate, have been represented. It may be seen how sample 1, from a system in which the bacteria usually appears, has a significantly higher signal than sample 2.

## Claims

1. An integrated filter-holder for filtering and cultivating a sample, comprising a top piece (1) with at least one upper hole (2) for an inlet flow to pass through and a bottom piece (3) with at least one lower hole (4) for an outlet flow to pass through, and which comprises at least one filtration membrane, and is **characterised in that**:
- it comprises an intermediate piece (5) that is arranged between the top piece (1) and the bottom piece (3) and which is joined to them, and in said intermediate piece (5) there is a reaction chamber (6) that is connected to the upper hole (2), and
- the filtration membrane is arranged between the intermediate piece (5) and the bottom piece (3).

2. The integrated filter-holder according to claim 1, **characterised in that** the reaction chamber (6) is a hollow space with a volume of less than 3 ml and is arranged over the filtration membrane.

3. The integrated filter-holder according to claim 2, **characterised in that** the volume of the filtration membrane is between 1 ml and 2 ml.

4. The integrated filter-holder according to claim 1, **characterised in that** it comprises two upper holes (2) in the top piece (1) and the reaction chamber (6) is joined to both.

5. The integrated filter-holder according to claim 1, **characterised in that** it comprises a cover linked to each one of the holes (2, 4).

6. The integrated filter-holder according to claim 1, **characterised in that** the filtration membrane is a porous membrane with a pore size comprised between 0.1 µm and 0.45 µm.

7. The integrated filter-holder according to claim 1, **characterised in that** it comprises a first groove (7) in the top piece (1) oriented towards the intermediate piece (5) which is intended to receive a first sealing gasket and a second groove (8) in the intermediate piece (5) oriented towards the bottom piece (3) which is intended to receive a second sealing gasket.

8. The integrated filter-holder according to claim 1, **characterised in that** the top (1), intermediate (5) and bottom (3) pieces are integrally joined together.

9. The integrated filter-holder according to claim 1 **characterised in that** it comprises a diffuser arranged in the intermediate piece (5) on the reaction chamber (6) and is a piece with a plurality of holes, configured to distribute the inlet flow from the at least one upper hole (2) towards the filtration membrane.

10. A microorganism concentration and detection method of a sample in an integrated filter-holder as described in any of claims 1 to 9, **characterised in that** it comprises the following stages:
- insert the sample through the top piece (1) making the sample pass through the filtration membrane arranged in the intermediate piece (5) such that the particles under study are retained in the filtration membrane,
- extract the filtered sample through the bottom piece (3),
- insert a solution through the top piece (1) into the reaction chamber (6) and keep it in the reaction chamber (6) for a predetermined period of time until it has reacted with the particles retained in the filtration membrane,
- extract the solution through the bottom piece (3),
- insert a reagent through the top piece (1) into the reaction chamber (6) and keep it in said reaction chamber (6) until it has reacted with the mixture of the solution and particles retained in the filtration membrane,
- extract the reagent through the bottom piece (3).

11. The concentration and detection method according to claim 10, **characterised in that** the reagent is kept inside the filter-holder for a period of time less than 20 minutes.

12. The concentration and detection method according to claim 10, **characterised in that** the sample is inserted through at least one upper hole (2) of the top piece (1) via a peristaltic pump configured to force the sample to pass through the filtration membrane and its release through the lower hole (4) of the bottom piece (3).

13. The concentration and detection method according to claim 10, **characterised in that** it is carried out in a filter-holder with two upper holes (2) and the sample, the solution and the reagent are inserted through an upper hole (2) and the solution and the reagent are extracted through the other upper hole.
